# FASCICULE DE BREVET EUROPEEN

(11) **EP 0 658 566 B1**
(45) Date de publication et mention de la délivrance du brevet: **02.07.1997**
(21) Numéro de dépôt: 94402879.4
(22) Date de dépôt: 14.12.1994
(51) Int. Cl.: C07H 21/00, B01J 19/00

(54) **Procédé de préparation de polynucléotides sur support solide et appareil permettant sa mise en oeuvre**
Verfahren zur Herstellung von Polynukleotiden auf einem Festträger und Apparat für dieses Verfahren
Process of polynucleotide preparation on a solid support and apparatus allowing it to be carried out

(30) Priorité: 16.12.1993 FR 9315164
(43) Date de publication de la demande: 21.06.1995
(73) Titulaire: GENSET, 75011 Paris (FR)
(72) Inventeur: Chatelain, François, Mountain View, CA 94040 (US); Kumarev, Viktor, F-93250 Villemonble (FR)
(74) Mandataire: Warcoin, Jacques

(56) Documents cités:
- EP-A- 0 114 599
- EP-A- 0 130 739
- EP-A- 0 181 491
- EP-A- 0 375 278
- EP-A- 0 541 340
- WO-A-85/01224
- WO-A-91/17823
- WO-A-92/02535

## Description

La présente invention concerne un procédé de préparation de polynucléotides sur support solide. La présente invention concerne également un réacteur contenant un support solide et un dispositif incluant ce réacteur utiles dans le procédé de préparation de polynucléotides selon l'invention.

La synthèse de polynucléotides sur support solide est particulièrement utilisée dans les synthèses automatisées d'oligonucléotides d'ADN ou ARN. Dans la présente demande, on entend par "polynucléotides" des fragments d'acides désoxyribonucléiques ou d'acides ribonucléiques, ou, plus généralement, des polynucléotides ou oligonucléotides où les bases, liens phosphates inter-nucléotidiques, ou encore les cycles riboses des bases, peuvent être modifiés chimiquement de façon connue. Il peut s'agir notamment d'oligonucléotides d'anoméries α ou β, d'oligonucléotides de lien internucléotidique du type phosphorothioate ou méthyl phosphonate, ou encore d'oligothionucléotides.

Le but de l'invention est l'amélioration des performances des méthodes existantes de synthèse d'oligonucléotides.

La méthode selon la présente invention consiste en une modification des appareils et paramètres de la synthèse organique afin d'en améliorer la productivité.

Les améliorations s'exercent, en particulier, sur la durée et les consommations de réactifs nécessaires à l'exécution d'une synthèse.

Le principe de la synthèse chimique des acides nucléiques sur support solide est, aujourd'hui, largement décrit dans la littérature spécialisée, et un certain nombre d'appareils sont disponibles sur le marché qui exécutent automatiquement tout ou partie des étapes de la synthèse. Parmi les voies chimiques décrites, seule la méthode dite des phosphoramidites (*Caruthers et col : EP 0 035 719 B1* ) présente à ce jour une efficacité suffisante pour envisager la production à l'échelle industrielle des acides nucléiques.

La préparation d'oligonucléotides ou de polynucléotides est réalisée dans un réacteur contenant un support solide et comprend le traitement du support solide tel qu'un support polymérique inorganique par une série d'étapes successives, chacune des séries conduisant à l'addition d'un nouveau nucléotide sur le support. Les séries d'étapes successives, ou cycles de synthèse, sont effectuées autant de fois que le nécessite la fabrication d'un oligonucléotide ou d'un polynucléotide de longueur souhaitée.

Dans un procédé de synthèse de polynucléotides sur support solide, le support solide est constitué traditionnellement de billes de verre à porosité contrôlée (CPG) ou, plus généralement, de particules d'un polymère minéral ou organique fonctionnalisé.

Les techniques classiquement mises en oeuvre impliquent l'utilisation de huit réactifs différents comme supports solides, constitués par ledit polymère minéral ou organique fonctionnalisé, lié à un nucléoside dA, dT, dC, dG, rA, rC, rG ou U, selon que la séquence à préparer comporte comme premier déoxyribo- ou ribo-nucléotide dA, dT, dC, dG, rA, rC, rG ou U. Les constructeurs fournissent donc des réacteurs où l'un de ces nucléosides a été préalablement attaché au support. Selon que la séquence commence par A, T, C, G, ou U, on choisit donc le réacteur approprié. L'élongation à partir de ce premier nucléoside se fait ensuite dans le sens 3' ―> 5', ou 5' ―> 3', grâce à des réactifs de couplage.

De nombreux supports ont déjà été décrits dans la littérature pour la synthèse en phase solide d'oligonucléotides.

On cite des polymères organiques tels que polystyrène (Nucleic Acids Res. (8), p 5507 (1980)), la polyacrylamide acryloylmorpholide, le polydiméthylacrylamide polymérisé sur gel de silice naturel (kieselguhr) (Nucleic Ac. Res. 9(7) 1691 (1980)).

D'autres supports décrits sont de nature inorganique, en particulier à base de silice fonctionnalisée par un radical hydrocarboné portant un groupe NH₂ et/ou COOH (*J.AM. Chem., 105, 661* (*1983* ), ou le support à base de silice fonctionnalisée par un groupement 3-aminopropyltriéthoxysilane dont l'utilisation en synthèse phosphite et phosphoramidite pour la préparation d'oligonucléotides a été décrite pour la première fois dans le brevet européen n° 0035719.

Il est connu, dans FR 93 08498, et PCT/FR94/00842, un procédé de synthèse d'oligonucléotides en phase solide dans lequel on utilise un support dit "universel", c'est-à-dire un support solide que l'on peut utiliser quel que soit le premier nucléotide de l'ARN ou ADN à synthétiser, quel que soit le type de réactif monomère utilisé pendant la synthèse, c'est-à-dire quel que soit le type de substitution sur le groupement phosphate en 3', ou en 5' selon que l'on fait la synthèse dans le sens 5' ―> 3', ou 3' ―> 5'.

En particulier, on peut obtenir "l'universalité" des supports en, phase solide grâce à une fonctionalisation du polymère minéral ou organique avec un radical hydrocarboné comportant des groupes du type glycol dans lesquels un groupe OH et un groupe nucléophile se trouvent en position vicinale, c'est-à-dire sur deux carbones adjacents, à l'extrémité du radical hydrocarboné, ces deux carbones pouvant être éventuellement substitués par des groupes inertes. On entend ici par "groupe inerte" un groupe qui ne réagit pas dans les conditions rencontrées lors des différentes étapes de la synthèse sur support solide d'acides nocléiques selon l'invention.

Dans un mode de réalisation particulier, un procédé de synthèse de polynucléotides comprend les étapes suivantes de :
1) condensation du groupe OH en 5' ou 3' du premier nucléotide ou d'un oligonucléotide relié à son autre extrémité 3' ou 5' au dit support solide, à l'aide d'un agent de couplage, avec le groupement phosphate éventuellement substitué respectivement en position 3' ou 5' d'un réactif monomère nucléotidique protégé en 3' et 5' ;
2) oxydation ou sulfuration du lien internucléotidique du type phosphite obtenu à l'étape 1) en un lien phosphate respectivement ;
3) déprotection de l'extrémité 5'-O ou 3'-O du produit obtenu à l'étape 2);
4) répétition des étapes 1) à 3) autant de fois que de nucléotides à ajouter pour synthétiser l'acide nucléique.

Les étapes ci-dessus conduisent à un oligonucléotide relié au support solide. Le procédé comporte une étape finale de décrochage de l'acide nucléique du support et élimination des groupes protecteurs des bases et, le cas échéant, des positions 2'-O de l'acide nucléique.

Dans les techniques où le support solide est déjà relié à un premier nucléoside correspondant au premier nucléotide de la séquence à préparer, avant le commencement des cycles de synthèse, ledit support comporte en général une protection en 5' ou 3' dudit nucléoside. Dans ce cas, le cycle de synthèse commence par une étape de déprotection en milieu acide, en général une détrylylation.

Selon les variantes les plus couramment utilisées, ledit réactif monomère nucléotidique répond à la formule : 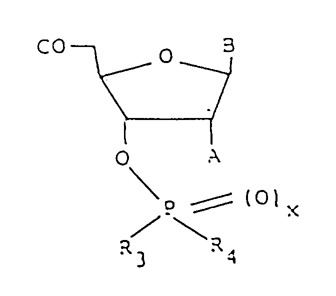 dans laquelle :
- A représente un atome d'hydrogène ou un groupement hydroxyle éventuellement protégé,
- B est une base purique ou pyrimidique dont la fonction amine exocyclique est éventuellement protégée,
- C est un groupe protecteur conventionnel de la fonction 5'-OH,
- x = 0 ou 1 avec
   a) lorsque x = 1 :
      - R₃ représente un atome d'hydrogène et R₄ représente un atome d'oxygène chargé négativement, ou
      - R₃ représente un atome d'oxygène et R₄ représente soit un atome d'oxygène, soit un atome d'oxygène porteur d'un groupe protecteur, et
   b) lorsque x = 0, R₃ est un atome d'oxygène porteur d'un groupe protecteur et R₄ est soit un halogène, soit un groupe amine disubstitué.

- Lorsque x est égal à 1, et R₃ est un atome d'oxygène, si R₄ est un atome d'oxygène, il s'agit de la méthode dite aux phosphodiesters ; si R₄ est un atome d'oxygène porteur d'un groupement protecteur, il s'agit de la méthode dite aux phosphotriesters.
- Lorsque x est égal 1, R₃ est un atome d'hydrogène et R₄ est un atome d'oxygène chargé négativement ; il s'agit alors de la méthode dite aux H-phosphosphonates.
- Lorsque x est égal à 0 et R₃ est un atome d'oxygène porteur d'un groupement protecteur, si R₄ est un halogène, il s'agit de la méthode dite des phosphites ; si R₄ est un groupe partant du type amine disubstitué, il s'agit de la méthode dite des phosphoramidites.

Les étapes d'un cycle de synthèse par la méthode aux phosphoramidites sont classiquement les suivantes:
1) condensation de l'hydroxyle 5'-terminal d'un nucléoside ou d'un oligonucléotide fixé de façon covalente au support solide avec un composé de type phosphite suivant la réaction: 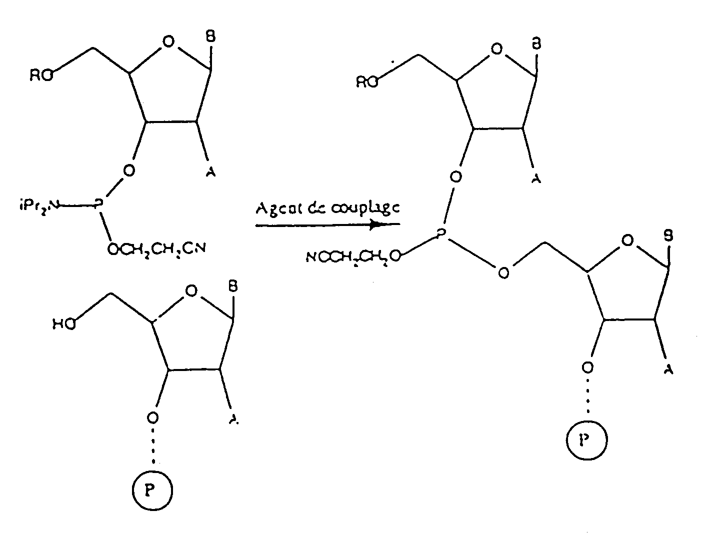
2) oxydation de la liaison phosphite obtenue en phosphate suivant la réaction: 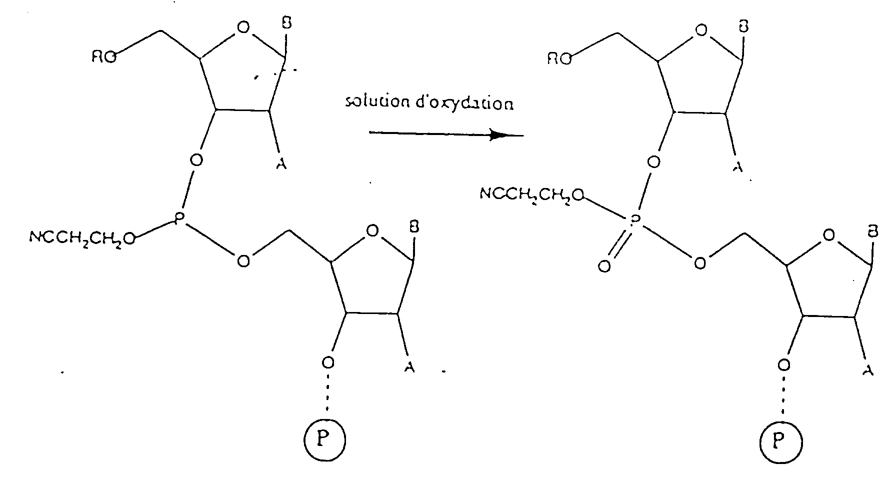
3) blocage des groupements hydroxyles des nucléosides n'ayant pas réagi;
4) libération de l'hydroxyle 5'-terminal du dernier nucléoside afin de générer un site actif pour le cycle de synthèse suivant.

Chaque nucléotide est ajouté séquentiellement sur le support par répétition des étapes 1 à 4. A la fin de la synthèse, l'oligonucléotide est séparé du support et libéré de ses groupements protecteurs par une réaction d'hydrolyse ménagée.

Les synthétiseurs commerciaux spécialisés dans la synthèse d'oligonucléotides sont conçus afin de réaliser automatiquement les étapes de synthèse ci-dessus décrites. Ces synthétiseurs sont composés généralement d'un réacteur contenant le support solide, d'un mélangeur de réactifs, d'un (ou plusieurs) bloc(s) de sélection des réactifs et des récipients contenant lesdits réactifs. Les étapes de la synthèse sont effectuées par l'addition successive des réactifs sélectionnés dans le réacteur. Le plus souvent, le support solide est lavé par de l'acétonitrile après chaque étape.

Le support solide n'est pas immobilisé et il n'occupe pas le volume entier du réacteur mais, en général, la moitié seulement du volume du réacteur, et en tout cas pas plus des trois-quarts, de manière à permettre une bonne agitation de la phase solide.

Le réacteur, tel qu'il est utilisé sur les synthétiseurs commerciaux, a une forme de récipient traversé par le flux des réactifs qui provoque l'agitation (ou la fluidisation) du support solide. On considère en effet que l'agitation de la phase solide est essentielle car elle permet une meilleure pénétration des solvants et réactifs dans les pores de la phase solide constituée en général de billes poreuses ou de membranes poreuses (voir *Methods in Molecular Biology : Protocols for Oligonuclcotides and Analogs* - *Synthesis and Properties - Edited by Sudhir Agrawal - 1993 - Humana Press, Totowa, New Jersey, pages 442-444 et 454* ).

Le mélangeur est situé en amont du réacteur, et relié à celui-ci par une canalisation. Les blocs de sélection des réactifs sont composés généralement d'électrovannes et permettent l'ouverture sélective des entrées/sorties du circuit hydraulique ainsi que des voies de cheminement des réactifs. La cohésion du système hydraulique est assurée par une série de tubes ou capillaires connectés de manière adéquate. Il est recommandé d'installer l'appareillage dans un local climatisé à 20°C, température à laquelle les réactions sont conduites.

Si l'efficacité des réactions est suffisante dans ces conditions, la durée des réactions et l'excès nécessaire de réactifs pour effectuer un bon lavage par dilutions successives, sont les principaux inconvénients des synthétiseurs tels que décrits ci-dessus. En l'occurrence, les consommations de réactifs et la durée des synthèses sont très supérieures aux valeurs calculées sur la base des lois connues de la synthèse organique. Ces inconvénients étaient attribués au facteur limitant que constitue la vitesse de diffusion des réactifs dans les pores.

On a découvert selon la présente invention que c'est principalement la géométrie du réacteur de type récipient, dans lequel les particules libres du support solide sont agitées selon le principe de réaction en phase "pseudo-liquide", qui comporte de nombreux inconvénients.

On a également découvert que plusieurs autres paramètres sont également en cause, qui diminuent considérablement la productivité des synthétiseurs:
- les réactifs utilisés pour l'étape d'oxydation (solution iode, tétrahydrofurane, pyridine, eau) se dégradent au cours du temps, et la baisse de réactivité consécutive induit rapidement une diminution de l'efficacité de la synthèse ;
- l'absence de thermostatisation du réacteur et des réactifs conduit à la perte de reproductibilité des synthèses.

Le système hydraulique lui-même comporte souvent des volumes morts non-utilisables. Le temps passé par les réactifs dans ces volumes morts et dans la chambre de mélange, en particulier lors de l'activation du phosphoramidite par l'agent de couplage, limite la réactivité des espèces transitoires formées.

C'est pourquoi la présente invention concerne un ensemble de modifications apportées au système ci-dessus décrit, ayant pour effet l'amélioration de la productivité par le biais de la diminution des consommations de réactifs et des durées de synthèse.

La caractéristique essentielle du procédé selon la présente invention se rapporte à l'immobilisation de la phase solide ou au mode de remplissage du réacteur par la phase solide.

La présente invention a en effet pour objet un procédé de préparation de polynucléotides sur support solide dans un réacteur en forme de colonne à travers laquelle on fait circuler les solutions de réactifs et/ou solvants, caractérisé en ce que la phase solide constituant ledit support solide est immobilisée dans ledit réacteur, et lesdites solutions migrent dans la colonne et à travers la phase solide selon une progression frontale, de sorte que les solutions successives de chaque étape d'un cycle de synthèse ne se mélangent pas.

La présente invention a également pour objet un procédé de préparation de polynucléotides sur support solide dans un réacteur en forme de colonne à travers laquelle on fait circuler les solutions de réactifs et/ou solvants, caractérisé en ce que le réacteur est une colonne entièrement remplie de particules d'un matériau poreux constituant ledit support solide.

Dans ce mode de réalisation où le support solide est constitué de particules libres d'un matériau poreux remplissant une colonne, les particules sont également immobilisées. L'immobilisation résulte en effet de ce qu'on entend ici par "colonne entièrement remplie" : le taux de remplissage en particules et la densité de répartition des particules dans la colonne sont tels que les particules sont suffisamment tassées de sorte qu'elles ne peuvent plus se mouvoir lorsque la colonne est traversée par un liquide.

Il peut s'agir d'une partie seulement de la colonne qui soit entièrement remplie, pourvu que les particules du support solide soient maintenues par des moyens appropriés dans ladite partie de la colonne.

Dans un mode de réalisation avantageux, la colonne aura une forme cylindrique.

On propose ainsi, pour la première fois, d'utiliser un réacteur de type "colonne de chromatographie", c'est-à-dire une colonne constituée d'un tube cylindrique entièrement rempli par la phase solide.

Ce type de colonne, remplie de manière homogène avec le support solide, offre plusieurs avantages: les différentes solutions qui se succèdent dans le réacteur ne se mélangent pas, ou peu : il n'y a donc pas de phénomène de dilution des solutions par le réactif précédent et le lavage consécutif à chaque étape du cycle de synthèse s'en trouve très facilité. Selon le principe des colonnes de chromatographie, les solutions successives introduites à une extrémité de la colonne migrent selon une progression frontale, de sorte qu'une solution "pousse" la précédente.

On a en effet observé que les phases solides utilisées classiquement en synthèse oligonucléotidique sur phase solide sont telles que l'affinité et les interactions physico-chimiques des différents réactifs et solvants utilisés sont négligeables et n'interfèrent pas sur leur diffusion à travers la phase solide autrement par les réactions chimiques souhaitées.

Il convient de mentionner que, bien entendu, dans la zone d'interface entre les solutions, il peut se produire localement un peu de mélange entre les solutions quand les débits de circulation des solutions sont importants. Toutefois ce mélange, localisé à l'interface, est sans commune mesure avec ce qui est observé dans les procédés antérieurs pour lesquels les lavages étaient basés sur le principe de dilution par mélange des solutions successives.

Contrairement au modèle classique, ces réacteurs permettent donc de diminuer notablement les volumes de réactifs nécessaires. Les débits utiles sont ajustés en fonction des constantes de diffusion et de la cinétique des réactions mises en jeu. La durée des cycles de synthèse s'en trouve considérablement diminuée. Pour chaque type de particule, en fonction de sa porosité, le débit des réactifs à travers la colonne ne doit pas dépasser une certaine valeur au-delà de laquelle les réactifs ne diffusent plus à l'intérieur des pores. Il s'agit d'un phénomène bien connu en chromatographie.

En outre, au sein du réacteur et quelle que soit sa taille, les conditions sont localement toujours identiques. Le système peut donc être appliqué à toutes les échelles de synthèse.

En particulier, on cite les matériaux constitués de polymères inorganiques, notamment de verre, de silice, d'oxydes métalliques, ou de polymères organiques, notamment de la cellulose, ou du polystyrène éventuellement substitué.

De préférence, le polymère est un polymère minéral constitué à base de verre ou de silice, notamment un gel de silice.

Lesdites particules peuvent être constituées de microbilles ou de microfibres agglomérées.

De préférence, dans le réacteur selon la présente invention, on utilise comme particules du support solide des microbilles poreuses, ce qui fournit la surface fonctionnalisée la plus importante en termes de poids de nucléosides liés au polymère par poids de polymère. La taille des billes peut aller de 5 µm à 500µ, plus généralement de 40 µm à 70 µm. Les pores peuvent éventuellement traverser de part en part les microbilles. Le diamètre des pores peut varier entre 200 Å et 10.000 Å. On peut avoir recours à deux tailles de pores : de larges pores (environ 10.000 Å) et des pores plus petits (300 Å). Cette association de pores de tailles variables favorise la diffusion des solutions à travers la phase solide.

Le réacteur selon la présente invention permet d'exploiter au mieux les lois connues de la diffusion et de la cinétique des réactions chimiques bimoléculaires selon lesquelles les réactifs en solution diffusent très rapidement dans les pores du support solide et la cinétique de condensation du nucléoside libre sur l'extrémité 5'OH de l'oligonucléotide fixé au support est quasi-instantanée.

Le contrôle de la température du réacteur et des réactifs est une autre caractéristique essentielle de la présente invention qui n'existe, à ce jour, sur aucun synthétiseur du marché.

La température de travail est l'un des paramètres qui influencent directement et simultanément la cinétique des réactions chimiques mises en jeu, mais aussi la stabilité des réactifs et la viscosité des solutions.

A l'encontre de tous les préjugés qui prétendent que la synthèse des oligonucléotides perd en efficacité au-dessus de 30°C, on préconise selon la présente invention de thermostater le réacteur à une température optimale de 45°C. La maîtrise de la température du système permet d'agir sur la cinétique des réactions chimiques mises en jeu ainsi que sur la viscosité des solutions utilisées.

De façon avantageuse, on mélange les réactifs, c'est-à-dire les agents de couplage tels que tétrazole, et les réactifs monomères, d'une part, et/ou les différents réactifs de la solution d'oxydation, d'autre part, immédiatement en amont du réacteur ou, éventuellement, juste en amont d'un système de distribution des réactifs dans un système multicolonne. De la sorte, on réduit les volumes morts non utilisables.

En particulier, on chauffe les réactifs avant de les introduire dans le réacteur et, le cas échéant, avant de les mélanger.

En effet, si on chauffe les réactifs seulement après leur mélange, on observe une inactivation mutuelle et une perte de réactivité sur la colonne. Plus précisément, le mélange précoce (trop loin du réacteur) des réactifs peut conduire à des réactions secondaires gênantes. En particulier, le mélange du tétrazole (acide faible) et des phosphoramidites peut entrainer la détritylation d'une partie des monomères. Les phosphoramidites peuvent ensuite réagir les uns avec les autres. Ce type de réaction conduit à la formation d'homopolymères qui peuvent ou non réagir avec l'oligonucléotide fixé au support. On peut constater, dans ce cas, la formation d'oligonucléotides plus longs que souhaité. Quoi qu'il en soit, le rendement de synthèse en est toujours diminué. Bien évidemment, ces phénomènes sont accentués par le chauffage. Il convient donc de chauffer les réactifs avant de les mélanger.

Dans les réacteurs utilisés avant la présente invention, au-dessus de 30°C les réactifs se dégradent rapidement. Mais, grâce au réacteur selon la présente invention, qui favorise la cinétique des réactions, il est possible d'augmenter la température jusqu'à 90°C (entre 20°C et 90°C) ; on règle de préférence la température du réacteur entre 30°C et 60°C, notamment à 45°C.

De manière avantageuse, l'étape de déprotection en milieu acide se fait avec du TFA (acide trifluoroacétique) dans du dichloroéthane. On utilise notamment une solution 1% v/v d'acide trifluoroacétique dans le dichloroéthane extra-sec, lors de l'étape de libération de la fonction 5' hydroxyle terminale (étape 4), en remplacement de la solution standard (2% d'acide trichloroacétique dans le dichlorométhane). Cette recommandation permet en particulier de s'affranchir des phénomènes d'ébullition éventuels du dichlorométhane, lorsque la température de travail excède 40°C.

Lors de l'étape d'oxydation, le mélange de l'acide acétique et de la pyridine provoque une augmentation des températures de 40°C à 60°C, qui facilite l'entretien de la température aux niveaux recommandés.

Grâce aux rendements de couplage très élevés (vraisemblablement supérieurs à 99%) du procédé selon l'invention, l'étape de blocage ( ou "capping") des fonctions 5'-OH n'ayant pas réagi (étape 3) s'avère, dans la plupart des cas, peu justifiée, voire complètement inutile et peut donc être omise. Ainsi, on ne remarque pas d'accumulation des fragments de synthèse de taille n-1 comme on pourrait s'y attendre lorsqu'on n'effectue pas l'étape de "capping". Vraisemblablement, lorsque l'efficacité des réactions est optimale, les espèces qui n'ont pas réagi sont définitivement inaccessibles.

Selon une autre caractéristique de la présente invention l'étape d'oxydation se fait avec de l'iode en solution dans l'acide acétique et de la pyridine. Ainsi, en lieu et place de la solution d'oxydation classique, on utilise séparément deux réactifs, l'un constitué par exemple, par une solution d'iode saturée dans l'acide acétique glacial, l'autre étant de la pyridine ultra-pure. Ces réactifs sont avantageux en ce que, à l'étape d'oxydation (étape 2 ci-dessus), ces deux solutions peuvent être mélangées en quantités stochiométriques à l'entrée du réacteur. Le mélange ainsi formé est injecté dans le réacteur et assure instantanément et quantitativement l'oxydation de la liaison internucléotidique nouvellement formée. Ainsi, on tire avantage de la stabilité des deux solutions séparées en garantissant indéfiniment la reproductibilité de l'étape d'oxydation.

La présente invention a également pour objet un réacteur pour préparer des polynucléotides selon un procédé de l'invention, le réacteur ayant une forme de colonne contenant un support solide à travers lequel on fait circuler les solutions de réactifs et/ou solvants, caractérisé en ce que la phase solide constituant le support solide est immobilisée dans ledit réacteur de sorte que lesdites solutions migrent dans la colonne et à travers ladite phase solide selon une progression frontale, les solutions successives de chaque étape d'un cycle de synthèse ne se mélangeant pas ou peu.

En particulier, la présente invention a donc pour objet un réacteur utile dans un procédé de préparation de polynucleotides sur support solide, caractérisé en ce qu'il est constitué d'une colonne cylindrique entièrement remplie par les particules de matériau poreux constituant le support solide tel que décrit ci-dessus.

La présente invention a enfin pour objet un dispositif pour la synthèse de polynucléotides sur support solide comportant un réacteur thermostaté, et un collecteur thermostaté qui réalise la collecte, le chauffage à la température du réacteur puis le mélange des réactifs avant leur introduction dans le réacteur.

D'autres avantages et caractéristiques de la présente invention apparaîtront à la lumière de la description détaillée qui va suivre.

Nous avons réalisé deux synthétiseurs d'oligonucléotides ou de polynucléotides conçus sur la base des améliorations techniques décrites ci-dessus. Les performances et la productivité de ces deux appareils s'en trouvent sensiblement améliorées par rapport à celles des appareils du marché.

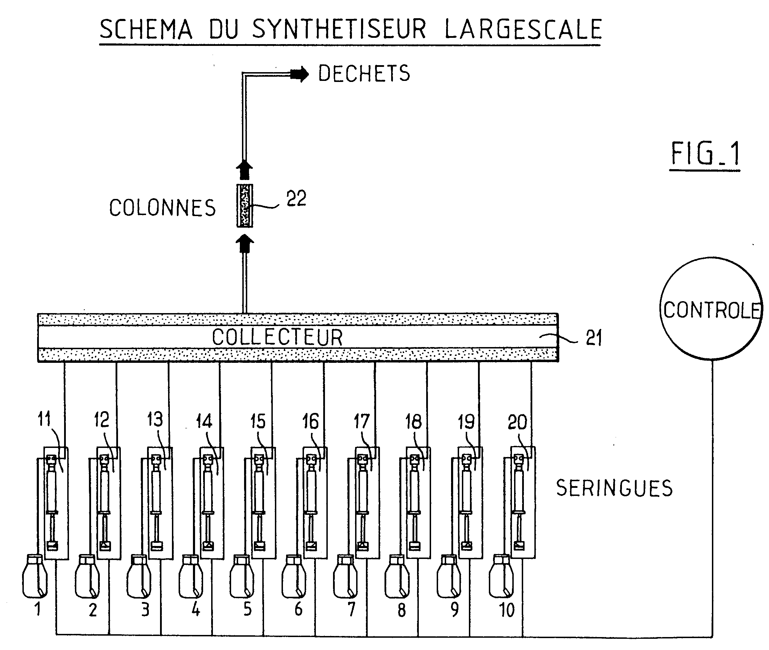

Le premier synthétiseur a été conçu pour synthétiser des quantités d'oligonucléotides comprises entre 50 umoles et 1 mmole. Ces performances peuvent donc être comparées à celles des synthétiseurs commerciaux, présents sur le marché, et capables de travailler à des échelles de synthèse équivalentes. Cet appareil est appelé "Synthétiseur Grande Echelle" (Large Scale). Il est représenté sur la Figure 1.

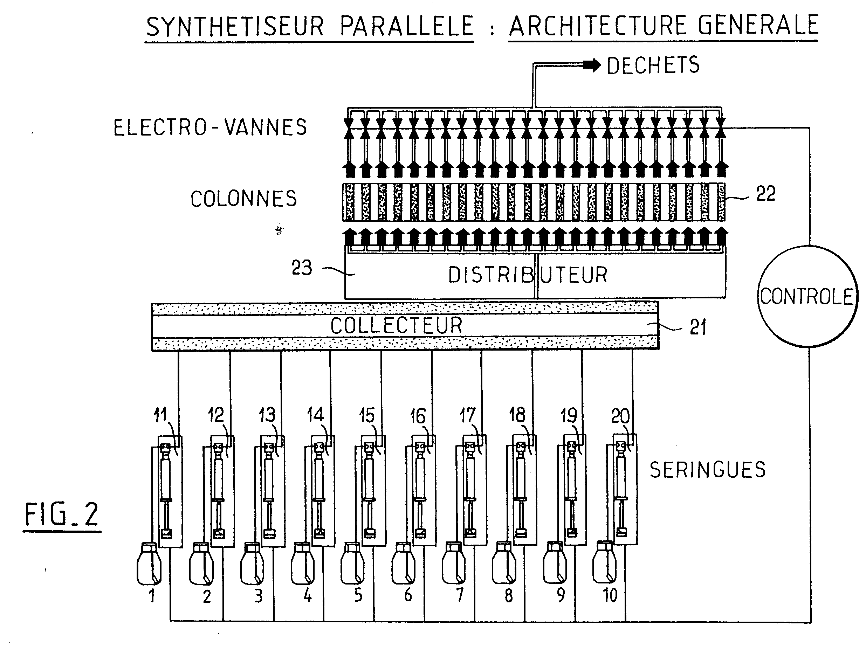

La fonction du second appareil est de synthétiser, en parallèle, 24 oligonucléotides ou polynucléotides différents. L'échelle de chaque synthèse est comprise entre 0,05 µmole et 1 µmole. A ce jour, cet appareil n'a pas d'équivalent. Sa productivité peut être rapprochée de celle de 6 synthétiseurs à 4 colonnes du marché. Il est appelé "Synthétiseur Multicolonne". Il est représenté sur la Figure 2.

Ces deux appareils comprennent:
- les récipients [(**1**) **à** (**10**)] contenant les réactifs, comportant chacun un module de pompage volumétrique de type seringue [(**11 à 20**)];
- un collecteur thermostaté [(**21**)] dont les attributs sont la collecte, le chauffage et le contrôle de la température, et le mélange des réactifs en mouvement.

Le "Synthétiseur Grande Echelle" est équipé d'un réacteur de type "colonne remplie" dont les dimensions sont ajustées en fonction de l'échelle de synthèse désirée [(**22**)].

Le "Synthétiseur Multicolonne" est équipé de 24 micro-colonnes [(**22**)] de même type que celle du "Synthétiseur Grande Echelle" mais dont les dimensions permettent de travailler aux échelles 0,05 µmole, 0,1 µmole, 0,2 µmole et 1 µmole. Entre le collecteur et les réacteurs se trouve un distributeur [(**23**)] chargé de répartir également les réactifs dans les 24 colonnes.

Enfin, un bloc composé de 24 électrovannes, asservies individuellement à chaque colonne, permet de sélectionner, à chaque étape de la synthèse, les réacteurs "en service".

Les volumes, les débits et les temps d'attente sont sous le contrôle d'un ordinateur. Le programme gère le bon enchaînement et l'exécution des cycles conformément aux séquences à synthétiser.

Afin de garantir des conditions locales toujours identiques quel que soit le nombre de réacteurs en service, le "Synthétiseur Multicolonne" fonctionne suivant un mode interactif. Ainsi, les volumes et les débits de chacun des réactifs sont ajustés, à chaque instant, au nombre de synthèses en cours.

### A - Déroulement d'une synthèse au phosphoramidite sur le "Synthétiseur Grande Echelle"

L'échelle de synthèse est choisie au moment du lancement du programme.

Chaque synthèse débute par un cycle d'initialisation qui permet de laver et de thermostater le réacteur à la température de travail.

Le programme comprend cinq cycles de synthèse correspondant à chacune des quatre bases et aux éventuelles bases modifiées.

Les cycles de synthèse se décomposent en étapes ou procédures de synthèse qui s'enchaînent dans l'ordre chronologique suivant:
1) Détritylation : libération de l'hydroxyle en position 5' du nucléoside ou de l'oligonucléotide fixé de façon covalente au support solide ;
2) Premier lavage ;
3) Addition de la base : condensation du monomère de type phosphoramidite sur l'hydroxy 5' terminal libre du nucléoside ou de l'oligonucléotide fixé au support solide ; formation de la liaison internucléotidique ;
4) Second lavage ;
5) Oxydation de la liaison internucléotidique de type phosphite précédemment formée en phosphate ;
6) Troisième lavage.

Ces cycles sont répétés autant de fois que le nécessite la synthèse d'un oligonucléotide ou d'un polynucléotide de la longueur souhaitée.

### B - Déroulement d'une synthèse sur le "Synthétiseur Multicolonne"

L'échelle de synthèse est choisie au moment du lancement du programme.

Chaque synthèse débute par un cycle d'initialisation qui permet de laver et de thermostater les réacteurs à la température de travail.

Il n'existe qu'un seul cycle de synthèse qui est répété autant de fois que le nécessite la fabrication des 24 oligo- ou polynucléotides.

Ce cycle peut être décomposé de la façon suivante :
a) Sélection des colonnes à détrityler.
   1) Détritylation : libération de l'hydroxyle en position 5' du nucléoside ou de l'oligonucléotide fixé de façon covalente au support solide.
   2) Premier lavage
b1) Sélection des colonnes devant recevoir la base A.
   3-1) Addition de la base A : condensation du monomère A de type phosphoramidite sur l'hydroxy 5' terminal libre du nucléoside ou de l'oligonucléotide fixé au support solide. Formation de la liaison internucléotidique.
b2) Sélection des colonnes devant recevoir la base G.
   3-2) Addition de la base G : condensation du monomère G de type phosphoramidite sur l'hydroxy 5' terminal libre du nucléoside ou de l'oligonucléotide fixé au support solide ; formation de la liaison internucléotidique.
b3) Sélection des colonnes devant recevoir la base T.
   3-3) Addition de la base T : condensation du monomère T de type phosphoramidite sur l'hydroxy 5' terminal libre du nucléoside ou de l'oligonucléotide fixé au support solide ; formation de la liaison internucléotidique.
b4) Sélection des colonnes devant recevoir la base C.
   3-4) Addition de la base C : condensation du monomère C de type phosphoramidite sur l'hydroxy 5' terminal libre du nucléoside ou de l'oligonucléotide fixé au support solide ; formation de la liaison internucléotidique.
c) Sélection des colonnes à oxyder.
   4) Second lavage.
   5) Oxydation de la liaison internucléotidique de type phosphite précédemment formée en phosphate.
   6) Troisième lavage.

### Description des étapes ci-dessus citées

Chaque étape peut être décrite en se référant aux schémas fournis des synthétiseurs (Figures 1 et 2).
1) Détritylation : le réactif n° 10, préférentiellement une solution d'acide trifluoroacétique à 1% v/v dans du dichloroéthane extra-sec, est poussé au travers du réacteur au moyen du module de seringue n° 10.
   Le volume nécessaire est compris entre 2 et 10 fois le volume du réacteur vide.
   Le débit dans la colonne est inférieur à 500 cm/min.
   Cette unité de débit en cm/min représente en fait la vitesse linéaire des solutions à l'intérieur de la colonne et est donc indépendante du diamètre de la colonne.
   Les débits en volume doivent être ajustés en fonction du diamètre du réacteur. Pour changer la taille du réacteur en conservant localement les conditions de synthèse, il suffit de modifier le débit en volume en faisant en sorte que la vitesse linéaire à l'intérieur du réacteur, donnée en cm/min, ne change pas.
   Les débits que l'on utilise sont compatibles avec les constantes de diffusion dans les pores, c'est-à-dire que la vitesse linéaire des solutions à l'intérieur de la colonne n'est pas supérieure à la vitesse de diffusion à travers les pores. Dans le cas contraire, on aurait une faible efficacité des réactifs et des lavages, conduisant à la diminution des rendements de synthèse.
   Le temps total de réaction est compris entre 10 et 120 secondes.
2) Premier lavage : le réactif n° 1, préférentiellement de l'acétonitrile extra-sec, est poussé au travers du réacteur au moyen du module de seringue n° 1.
   Le volume nécessaire est compris entre 2 et 10 fois le volume du réacteur vide.
   Le débit dans la colonne est inférieur à 500 cm/min.
3) Couplage : les réactifs n° 3, ou n° 4, ou n° 5, ou n° 6, ou n° 7, et n° 2, respectivement une solution 0,1 M de chaque monomère et une solution 0,45% de tétrazole dans l'acétonitrile, sont poussés conjointement au travers du réacteur au moyen des modules de seringue n° 3 ou n° 4 , ou n° 5, ou n° 6, ou n° 7, et n° 2 respectivement.
   Les rapports des volumes et des débits des réactifs n° 3, ou n° 4, ou n° 5, ou n° 6, ou n° 7, et n° 2 peuvent être différents de 1.
   Le volume total nécessaire est compris entre 2 et 10 fois le volume du réacteur vide.
   Le débit global dans la colonne est inférieur à 500 cm/min.
   Le temps de réaction est inférieur à 1 minute.
4) Second lavage : le réactif n° 1, préférentiellement de l'acétonitrile extra-sec, est poussé au travers du réacteur au moyen du module de seringue n° 1.
   Le volume nécessaire est compris entre 0,5 et 5 fois le volume du réacteur vide.
   Le débit dans la colonne est inférieur à 500 cm/min.
5) Oxydation : les réactifs n° 8 et n° 9, respectivement une solution saturée d'iode dans l'acide acétique glacial et de la pyridine ultra-pure, sont poussés conjointement au travers du réacteur au moyen des modules de seringue n° 8 et n° 9 respectivement.
   Les rapports des volumes et des débits des réactifs n° 8 et n° 9 sont égaux à 1.
   Le volume total nécessaire est compris entre 1 et 5 fois le volume du réacteur vide.
   Le débit global dans la colonne est inférieur à 500 cm/min.
   Le temps de réaction est inférieur à 30 secondes.
6) Troisième lavage : le réactif n° 1, préférentiellement de l'acétonitrile extra-sec, est poussé au travers du réacteur au moyen du module de seringue n° 1.
   Le volume nécessaire est compris entre 1 et 5 fois le volume du réacteur vide.
   Le débit dans la colonne est inférieur à 500 cm/min.

Les exemples de tailles de réalisation qui suivent servent à illustrer le procédé selon l'invention.

### Exemple 1 : Synthèse d'un oligonucléotide, à l'échelle 30 umoles, à l'aide du "Synthétiseur Grande Echelle"

L'appareil utilisé est celui décrit précédemment.

Le réacteur utilisé est une colonne cylindrique en verre, de diamètre 10 mm et de hauteur 25 mm.

La température de travail est de 45°C.

Les cycles de synthèse sont détaillés dans le Tableau 1.

On a synthétisé, dans ces conditions, un oligodéoxynucléotide, long de 18 bases, dont la séquence est: d(ACG TTC CTC CTG CGG GAA).

Le réacteur est rempli soigneusement avec 0,66 g de CPG 500Å (CPG INC., USA), "dérivatisé" par le premier nucléoside A. La capacité du support est de 45 umoles/g (densité de 3 ml/g).

L'échelle de synthèse est de 30 µmoles.

Après une étape de lavage par l'acétonitrile, on exécute 17 fois le cycle de synthèse tel qu'il est décrit dans le Tableau 1.

Dans ces conditions, l'oligonucléotide, de longueur souhaitée, conserve le groupement transitoire diméthoxytrityl en position 5' terminale.

L'oligonucléotide est séparé de la CPG et libéré des groupements protecteurs permanents par un traitement approprié du support "dérivatisé", suivant la méthode ci-dessus décrite, par 10 ml d'une solution aqueuse d'ammoniaque à 30%, pendant 16 heures à 55°C.

Après ajout de 40 ml d'éthanol absolu et 1 ml d'une solution aqueuse d'acétate de sodium 3M, l'oligonucléotide est mis à précipiter pendant deux heures à 0°C.

Le précipitat est ensuite filtré sur une membrane loprodyne de porosité 1,2 µm (PALL S.A., FRANCE) et resolubilisé dans 10 ml d'eau.

Après lecture de la densité optique à 260 nm, on obtient 4000 U.D.O., soit 120 mg de mélange brut de synthèse.

La pureté de l'oligonucléotide de longueur souhaitée, estimée par HPLC sur colonne de phase inverse, est de 88%.

### Exemple 2 : Synthèse d'un oligonucléotide, à l'échelle 77 µmoles, à l'aide du "Synthétiseur Grande Echelle"

L'appareil utilisé est le même que pour l'exemple précédent.

Le réacteur utilisé est une colonne cylindrique en verre, de diamètre 10 mm et de hauteur 25 mm.

La température de travail est de 45°C.

Les cycles de synthèse sont détaillés dans le Tableau 2.

On a synthétisé, dans ces conditions, un oligodéoxynucléotide, long de 18 bases, dont la séquence est : d(TTC CGC CAG GAG GAA CGT).

Le réacteur est rempli soigneusement avec 0,66 g de CPG 500Å Forte Capacité ("High-Loaded") (MILLIPORE S.A., FRANCE) "dérivatisé" par le premier nucléoside T. La capacité du support est de 110 µmoles/g, (densité de 3 ml/g).

L'échelle de synthèse est de 77 umoles.

Après une étape de lavage par l'acétonitrile, nous avons exécuté 17 fois le cycle de synthèse tel qu'il est décrit dans le Tableau 2.

Dans ces conditions, l'oligonucléotide, de longueur souhaitée, conserve le groupement transitoire diméthoxytrityl en position 5' terminale.

Les conditions de déprotection et de récupération de l'oligonucléotide sont les mêmes que celles décrités à l'exemple précédent.

Après lecture de la densité optique à 260 nm, on obtient 8500 U.D.O., soit 255 mg de mélange brut de synthèse.

La pureté de l'oligonucléotide de longueur souhaitée, estimée par HPLC sur colonne de phase inverse, est de 84%.

### Exemple 3 : Synthèse d'un oligonucléotide, à l'échelle 100 umoles, à l'aide du "Synthétiseur Grande Echelle"

L'appareil utilisé est le même que pour l'exemple précédent.

Le réacteur utilisé est une colonne cylindrique en verre, de diamètre 15 mm et de hauteur 34 mm.

La température de travail est de 45°C.

Les cycles de synthèse sont détaillés dans le Tableau 3.

On synthétise, dans ces conditions, un oligodéoxynucléotide, long de 56 bases, dont la séquence est : d(TAA CCA CAC TTT TTG TGT GGT TAA TGA TCT ACA GTT ATT TTT TAA CTG TAG ATC AT).

Le réacteur est rempli soigneusement avec 2 g de CPG 500 Å (MILLIPORE S.A., FRANCE), "dérivatisé" par le premier nucléoside T. La capacité du support est de 50 µmoles/g, (densité de 3 ml/g).

L'échelle de synthèse est de 100 µmoles.

Après une étape de lavage par l'acétonitrile, nous avons exécuté 55 fois le cycle de synthèse tel qu'il est décrit dans le Tableau 3.

Dans ces conditions, l'oligonucléotide, de longueur souhaitée, conserve le groupement transitoire dimétoxytrityl en position 5' terminale.

Les conditions de déprotection et de récupération de l'oligonucléotide sont les mêmes que celles décrites aux exemples précédents.

Après lecture de la densité optique à 260 nm, on obtient 31 000 U.D.O., soit 930 mg de mélange brut de synthèse.

La pureté de l'oligonucléotide de longueur souhaitée, estimée par HPLC sur colonne de phase inverse, est de 61%.

### Exemple 4 : Synthèses simultanées de 24 oligonucléotides différents à l'aide du "Synthétiseur Multicolonne"

Les réacteurs sont des micro-colonnes métalliques de diamètre 1,5 mm et de hauteur 6 mm.

La température de travail est de 50°C.

Les cycles de synthèse sont détaillés dans le Tableau 4.

Nous avons synthétisé, dans ces conditions, les oligodéoxynucléotides dont les séquences sont données dans le Tableau 5.

Les réacteurs sont remplis régulièrement avec 2 mg de CPG 500 Å universelle comportant un groupe époxyde (préparé selon l'exemple 1 du brevet FR 2707296) (GENSET S.A. FRANCE). La capacité du support est de 50 µmoles/g(densité : 3 ml/g).

L'échelle de synthèse est de 0,1 µmoles.

Après une étape de lavage par l'acétonitrile; nous avons exécuté les opérations décrites dans le Tableau 4, autant de fois que le nécessite la synthèse des 24 oligodéoxynucléotides du Tableau 5.

Les conditions de déprotection et de récupération de l'oligonucléotide sont les mêmes que celles décrites aux exemples précédents.

Après lecture de la densité optique à 260 nm, on obtient, en moyenne, 11 U.D.O. de chacun des oligonucléotides, soit 0,33 mg. La pureté des oligonucléotides, estimée par HPLC, sur colonne de phase inverse pour les oligodéoxynucléotides 5' O-Trityl, et sur colonne échangeuse d'anions pour les oligonucléotides 5'OH, excède 84%.

### Exemple comparatif 5 :Synthèses d'oligonucléotides à l'aide des réacteurs APPLIED BIOSYSTEMS et MILLIPORE

Le Tableau 6 ci-après présente les caractéristiques des synthèses effectuées avec des réacteurs à fluidisation commercialisés par APPLIED BIOSYSTEMS et MILLIPORE telles que décrites dans la littérature.

Les synthétiseurs utilisés sont les suivants:
- SYNTHETISEUR APPLIED BIOSYSTEMS, modèle 394 :
   - . réacteur en forme de colonne:: diamètre 5 mm
   hauteur 6 mm
   volume 0,11 ml
   - . support solide:: CPG 500 Å (CPG INC., USA)
   capacité: 30 µmoles/g
   densité: 3 ml/g
   - Pour une synthèse à l'échelle 0,2 µmole:: 6,7 mg de CPG, soit un volume de 0,02ml et un taux de remplissage de 20%.
- SYNTHETISEUR MILLIPORE modèle 8800:
   . Réacteur en forme de récipient de 225 ml, utilisable avec 1 à 15 g de CPG.
- Pour nos exemples:
   . méthode "STANDARD" pour 100 µmoles
      - . support solide:: CPG 500Å (MILLIPORE S.A., FRANCE) capacité: 30 µmoles/g densité: 3 ml/g
      - Soit pour 100 µmoles:: 3,33 g de CPG
      soit 10 ml de phase et un taux de remplissagee 4,5%.
   . méthode "AMELIOREE" pour 100 µmoles
      - . support solide:: CPG 500Å Forte Capacité ("High Load")
      MILLIPORE S.A., FRANCE)
      capacité: 100 µmoles/g
      densité: 3 ml/g
      - Soit pour 100 µmoles:: 1 g de CPG
      soit 3 ml de phase et un taux de remplissage de 1,3%.

En comparaison aux résultats décrits aux Exemples 1 à 4, la diminution de la durée de synthèse et des quaxitités de réactifs est considérable (Tableau 6).
Pour la synthèse de 100 µmoles :
- avec le procédé selon l'invention (Exemple 3 et Tableau 3), on utilise 118 ml de réatifs et solvants, et la durée de synthèse est de 3 minutes par cycle de synthèse ;
- avec une colonne MILLIPORE, on utilise 500 ml de réactifs et solvants, et la durée de synthèse est de 20 minutes par cycle.

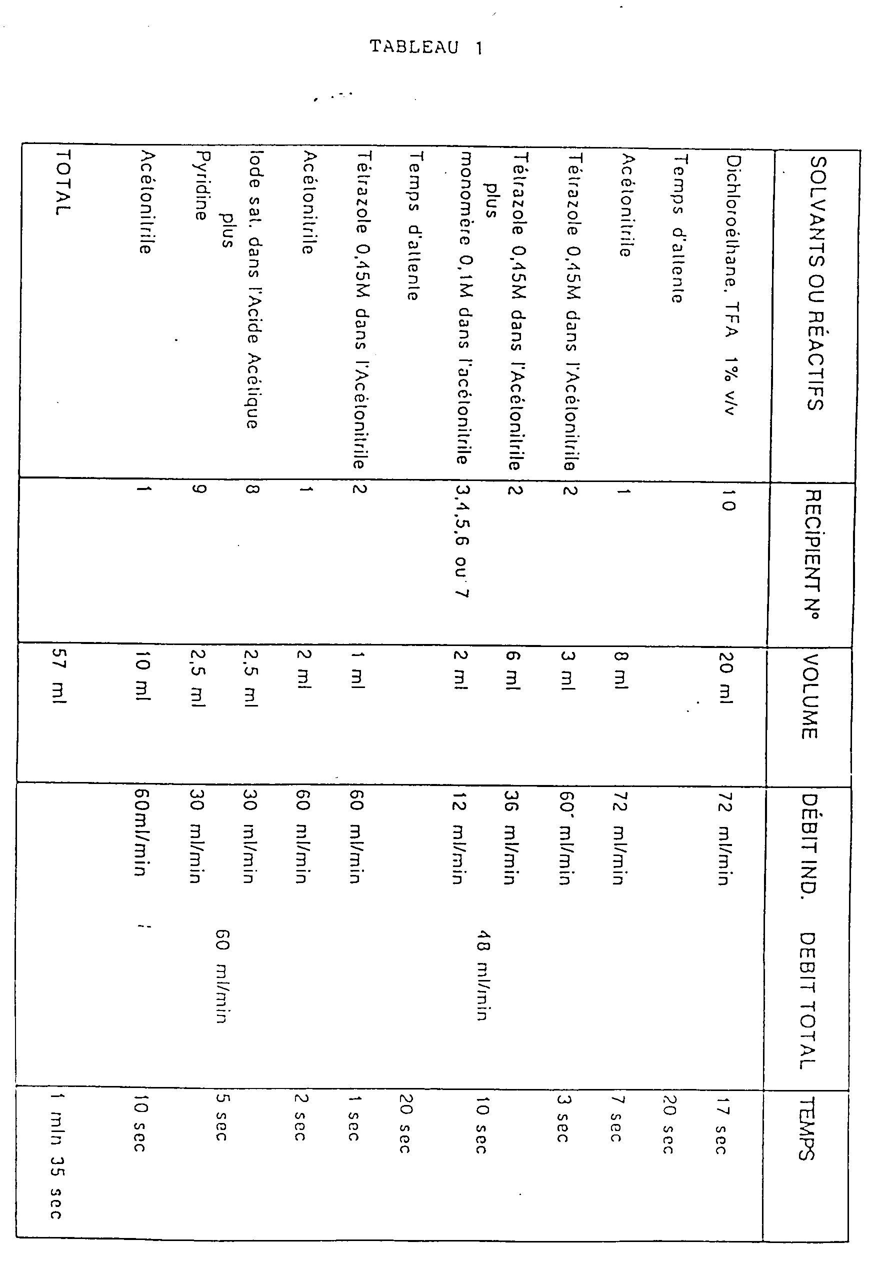

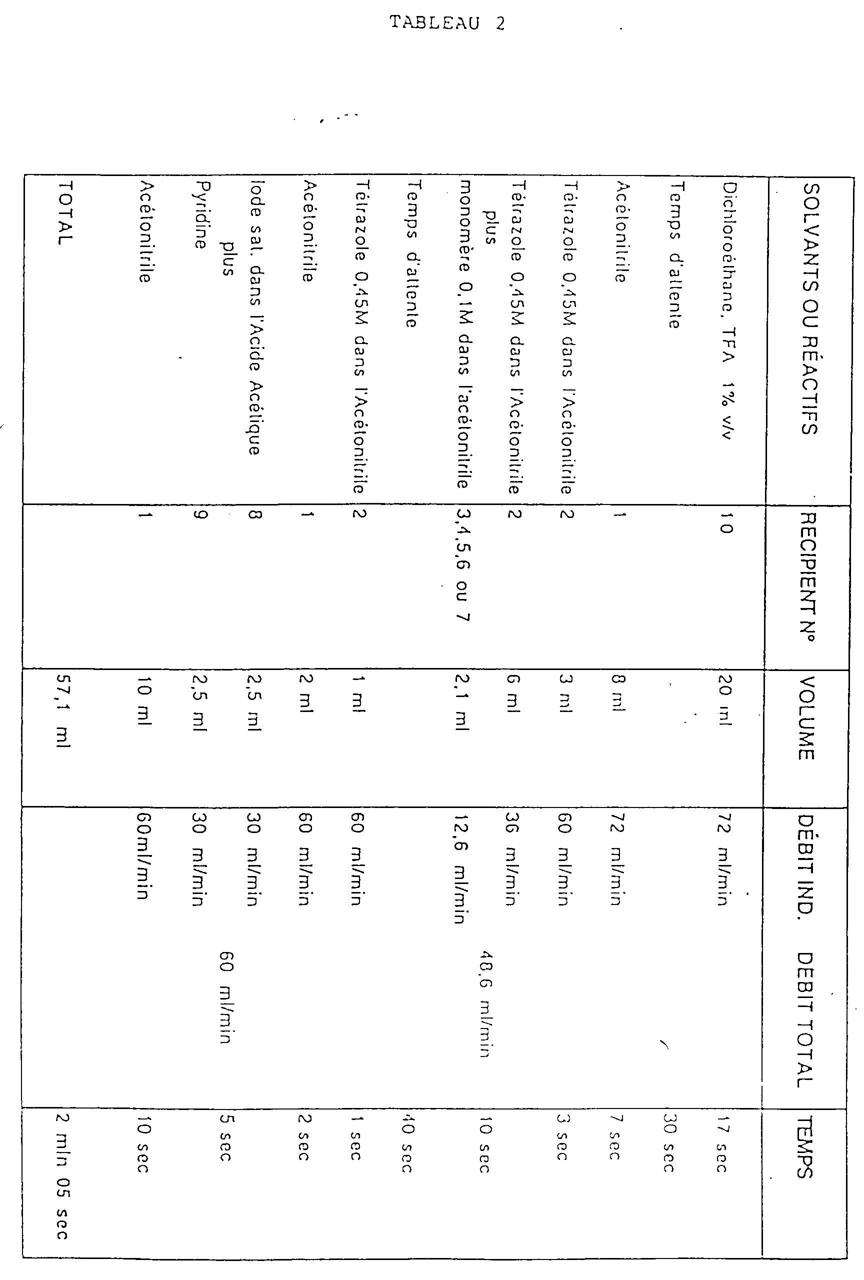

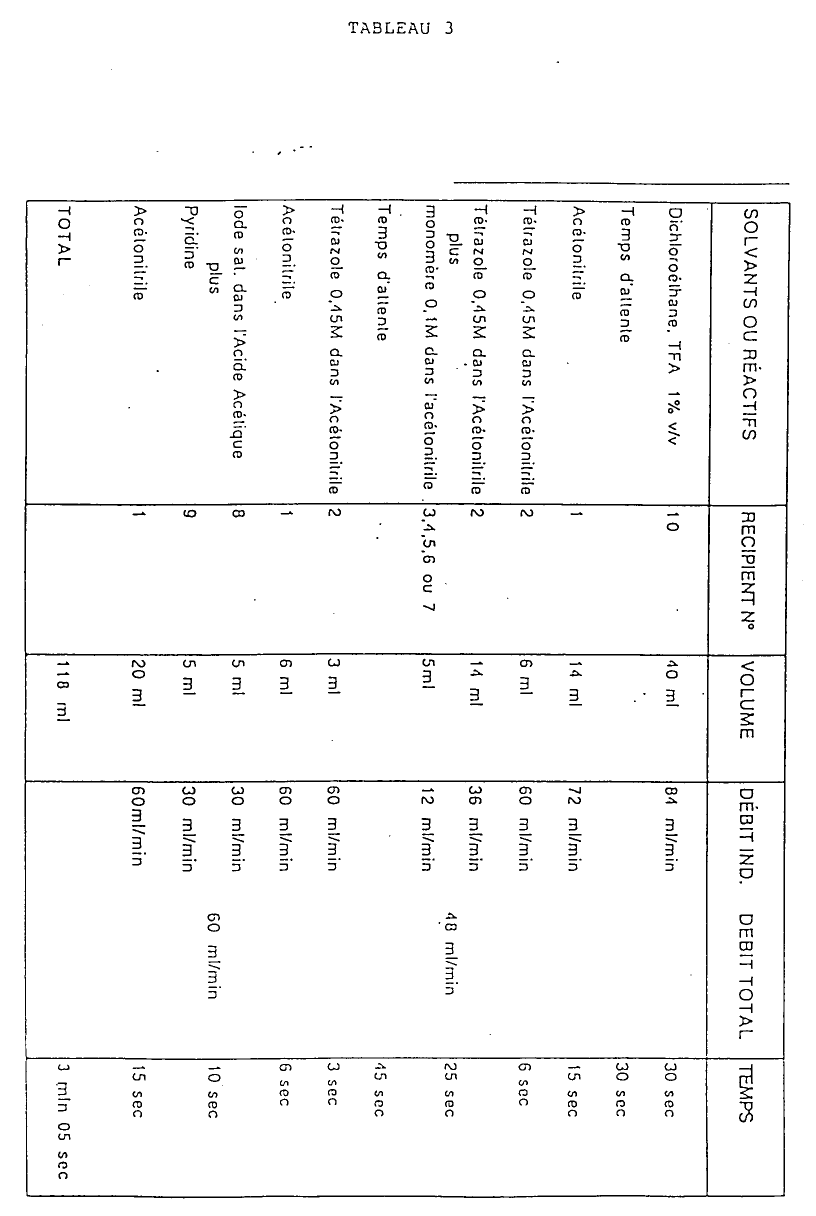

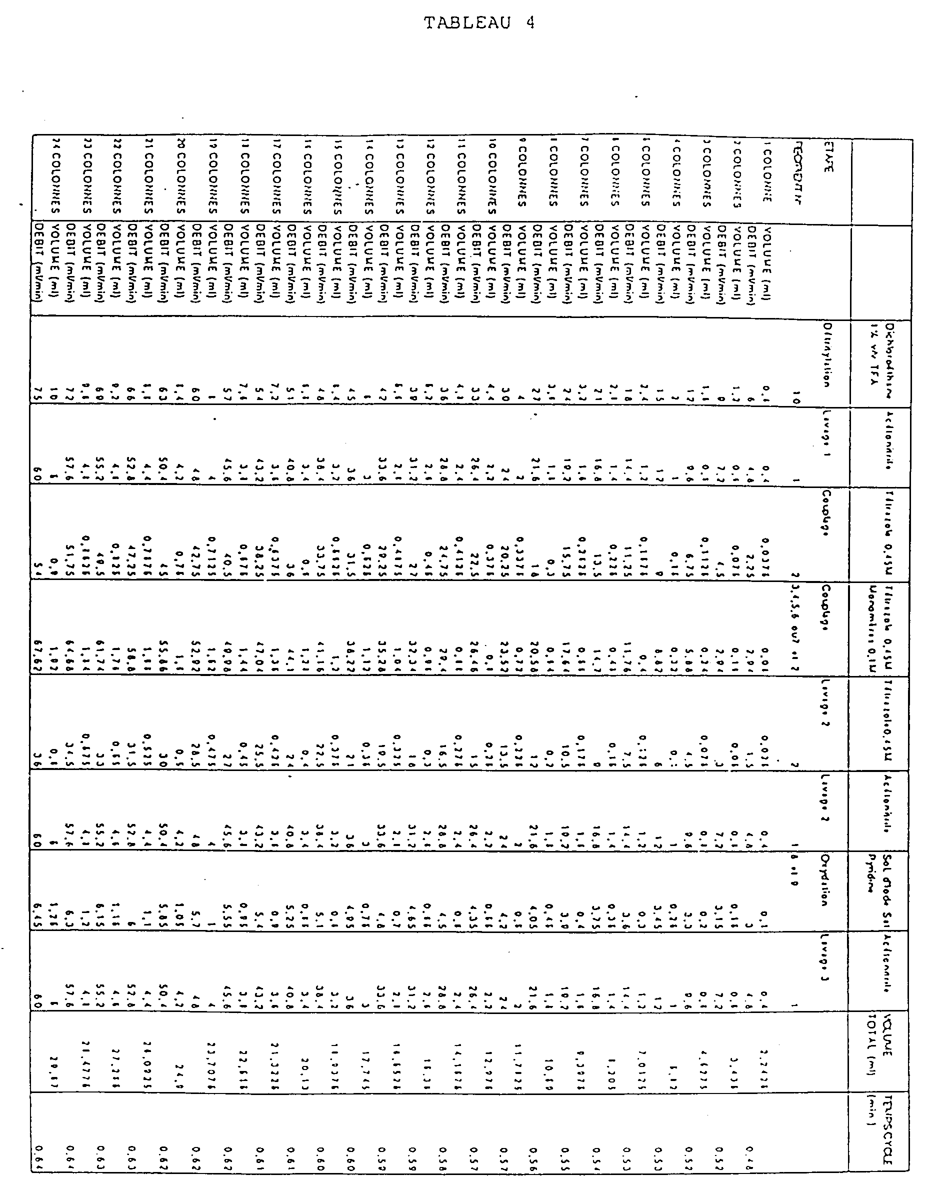

**TABLEAU 5**

| COLONNE N° | OLIGO | 5' Trltyl | Nbre bases | SEQUENCE 5'-3' |
|---|---|---|---|---|
| 0 | OLIGO1 | OUI | 12 | TTT TTT TTT TTT |
| 1 | OLIGO2 | NON | 12 | TTT TTT TTT TTT |
| 2 | OLIGO3 | OUI | 15 | TTT TTT TTT TTT TTT |
| 3 | OLIGO4 | NON | 15 | TTT TTT TTT TTT TTT |
| 4 | OLIGO5 | OUI | 18 | TTT TTT TTT TTT TTT TTT |
| 5 | OLIGO6 | NON | 18 | TTT TTT TTT TTT TTT TTT |
| 6 | OLIGO7 | OUI | 12 | AAA AAA AAA AAA |
| 7 | OLIGO8 | NON | 12 | AAA AAA AAA AAA |
| 8 | OLIGO9 | OUI | 15 | AAA AAA AAA AAA AAA |
| 9 | OLIGO10 | NON | 15 | AAA AAA AAA AAA AAA |
| 10 | OLIGO11 | OUI | 18 | AAA AAA AAA AAA AAA AAA |
| 11 | OLIGO12 | NON | 18 | AAA AAA AAA AAA AAA AAA |
| 12 | OLIGO13 | OUI | 12 | CCC CCC CCC CCC |
| 13 | OLIGO14 | NON | 12 | CCC CCC CCC CCC |
| 14 | OLIGO15 | OUI | 15 | CCC CCC CCC CCC CCC |
| 15 | OLIGO16 | NON | 15 | CCC CCC CCC CCC CCC |
| 16 | OLIGO17 | OUI | 18 | CCC CCC CCC CCC CCC CCC |
| 17 | OLIGO18 | NON | 18 | CCC CCC CCC CCC CCC CCC |
| 18 | OLIGO19 | OUI | 12 | AGT CAG TCA GTC |
| 19 | OLIGO20 | NON | 12 | AGT CAG TCA GTC |
| 20 | OLIGO21 | OUI | 15 | AGT CAG TCA GTC AGT |
| 21 | OLIGO22 | NON | 15 | AGT CAG TCA GTC AGT |
| 22 | OLIGO23 | OUI | 18 | AGT CAG TCA GTC AGT CAG |
| 23 | OLIGO24 | NON | 18 | AGT CAG TCA GTC AGT CAG |

**TABLEAU 6**

| SYNTHETISEURS CARACTERISTIQUES ECHELLE DE SYNTHESE METHODE | ABI 394 4 COLONNES 0,2 µmoles STANDARD^{·} | MILLIPORE LARGE-SCALE 100 µmoles STANDARD^{··} | MILLIPORE LARGE-SCALE 100 µmoles AMELIORE^{··} |
|---|---|---|---|
| REACTIFS | (ml) | (ml) | (ml) |
| MONOMERES 0,1M | 0,110 | 5 | 2,62 |
| SOLUTION DE TETRAZOLE | 0,400 | 20,3 | 19 |
| CAP A (anhydride acétique) | 0,290 | 9 | 6,25 |
| CAP B (N-Méthylimidazole) | 0,260 | 11,6 | 8,65 |
| DEBLOCK (TCA/DCM) | 1,100 | 84,3 | 68,75 |
| SOLUTION D'IODE | 0,350 | 40 | 33 |
| ACETONITRILE | 8,000 | 376 | 286,25 |
| TOTAL | 10,510 | 546,2 | 424,52 |
| TEMPS PAR CYCLE | 6 mln. | 20 mln. | 22 mln. |

| | | | |
|---|---|---|---|
| ^{·} APPLIED BIOSYSTEMS Manuel d'utilisation du synthétiseur ABI 394 | | | |
| ^{··} N.D.SINHA, S.FRY; 3rd CAMBRIDGE SYMPOSIUM Oligonucléotides & Analogues, 5-8 septembre 1993 POSTER COMMUNICATION | | | |

## Revendications

1. Procédé de préparation de polynucléotides sur support solide dans un réacteur en forme de colonne à travers laquelle on fait circuler les solutions de réactifs et/ou solvants, caractérisé en ce que la phase solide constituant ledit support solide est immobilisée dans ledit réacteur, et lesdites solutions migrent dans la colonne et à travers la phase solide selon une progression frontale, de sorte que les solutions successives de chaque étape d'un cycle de synthèse ne se mélangent pas.

2. Procédé de préparation de polynucléotides sur support solide dans un réacteur en forme de colonne à travers laquelle on fait circuler les solutions de réactifs et/ou solvants, caractérisé en ce que le réacteur est une colonne entièrement remplie de particules d'un matériaux poreux constituant ledit support solide.

3. Procédé selon la revendication 1 ou 2 caractérisé en ce que le réacteur a la forme d'une colonne cylindrique.

4. Procédé selon l'une des revendications 1 à 3 caractérisé en ce que la phase solide est constituée de particules consistant en des microbilles poreuses.

5. Procédé selon l'une des revendications 1 à 4 caractérisé en ce que le réacteur est maintenu à une température comprise entre 30°C et 60°C, de préférence de l'ordre de 45°C.

6. Procédé selon l'une des revendications 1 à 5 caractérisé en ce qu'on chauffe les réactifs à la température du réacteur avant de les introduire dans ledit réacteur et, le cas échéant, avant de les mélanger.

7. Procédé selon l'une des revendications 1 à 6 caractérisé en ce que l'étape de déprotection se fait avec du TFA dans du dichloroéthane.

8. Procédé selon l'une des revendications 1 à 7 caractérisé en ce que l'étape d'oxydation se fait avec de l'iode en solution dans l'acide acétique et de la pyridine.

9. Réacteur pour préparer des polynucléotides selon un procédé des revendications 1 à 8, le réacteur ayant une forme de colonne contenant un support solide à travers lequel on fait circuler les solutions de réactifs et/ou solvants, caractérisé en ce que la phase solide constituant le support solide est immobilisée dans ledit réacteur de sorte que lesdites solutions migrent dans la colonne et à travers ladite phase solide selon une progression frontale, les solutions successives de chaque étape d'un cycle de synthèse ne se mélangeant pas ou peu.

10. Réacteur utile dans un procédé de préparation de polynucléotides sur support solide selon l'une des revendications 1 à 8, caractérisé en ce qu'il est constitué d'une colonne cylindrique entièrement remplie par les particules de matériaux poreux constituant le support solide.

11. Réacteur selon la revendication 10 caractérisé en ce que les particules sont des microbilles poreuses.

12. Réacteur selon l'une des revendications 9 à 11, caractérisé en ce que le réacteur peut être thermostaté à une température entre 30°C et 60°C, de préférence 45°C.

13. Dispositif pour la synthèse de polynucléotides sur support solide comportant le réacteur thermostaté selon l'une des revendications 9 à 12, et un collecteur thermostaté qui réalise la collecte, le chauffage à la température du réacteur, puis le mélange des réactifs avant leur introduction dans le réacteur.

## Claims

1. Process for preparing polynucleotides on a solid support in a reactor in the form of a column through which solutions of reagents and/or solvents are circulated, characterized in that the solid phase constituting the said solid support is immobilized in the said reactor, and the said solutions migrate in the column and through the solid phase according to a frontal progression, such that the successive solutions from each step of a synthesis cycle do not mix.

2. Process for preparing polynucleotides on a solid support in a reactor in the form of a column through which the solutions of reagents and/or solvents are circulated, characterized in that the reactor is a column completely filled with particles of a porous material constituting the said solid support.

3. Process according to Claim 1 or 2, characterized in that the reactor has the shape of a cylindrical column.

4. Process according to one of Claims 1 to 3, characterized in that the solid phase consists of particles composed of porous microbeads.

5. Process according to one of Claims 1 to 4, characterized in that the reactor is maintained at a temperature of between 30°C and 60°C, preferably of the order of 45°C.

6. Process according to one of Claims 1 to 5, characterized in that the reagents are heated to the temperature of the reactor before introducing them into the said reactor and, where appropriate, before mixing them.

7. Process according to one of Claims 1 to 6, characterized in that the deprotection step is carried out with TFA in dichloroethane.

8. Process according to one of Claims 1 to 7, characterized in that the oxidation step is carried out with iodine dissolved in acetic acid and pyridine.

9. Reactor for preparing polynucleotides according to a process of Claims 1 to 8, the reactor being in the form of a column containing a solid support through which the solutions of reagents and/or solvents are circulated, characterized in that the solid phase constituting the solid support is immobilized in the said reactor such that the said solutions migrate in the column and through the said solid phase according to a frontal progression, the successive solutions of each step of a synthesis cycle not mixing at all or very little.

10. Reactor which is useful in a process for preparing polynucleotides on a solid support according to one of Claims 1 to 8, characterized in that it consists of a cylindrical column completely filled with particles of porous materials constituting the solid support.

11. Reactor according to Claim 10, characterized in that the particles are porous microbeads.

12. Reactor according to one of Claims 9 to 11, characterized in that the reactor can be thermostatted at a temperature between 30°C and 60°C, preferably 45°C.

13. Device for the synthesis of polynucleotides on a solid support comprising the thermostatted reactor according to one of Claims 9 to 12, and a thermostatted collector which performs the collection, the heating to the temperature of the reactor, then the mixing of the reagents before their introduction into the reactor.

## Patentansprüche

1. Verfahren zur Herstellung von Polynucleotiden auf einem festen Träger in einem Reaktionsgefäß in Form einer Kolonne, durch die man die Lösungen der Reagentien und/oder Lösungsmittel zirkulieren läßt, dadurch gekennzeichnet, daß die feste Phase, die den genannten festen Träger darstellt, in dem genannten Reaktionsgefäß immobilisiert ist und daß die genannten Lösungen in der Kolonne und durch die feste Phase hindurch in Form einer fortschreitenden Frontlinie in der Weise wandern, daß die aufeinanderfolgenden Lösungen jeder Stufe eines Synthesezyklus sich nicht vermischen.

2. Verfahren zur Herstellung von Polynucleotiden auf einem festen Träger in einem Reaktionsgefäß in Form einer Kolonne, durch die man die Lösungen der Reagentien und/oder Lösungsmittel zirkulieren läßt, dadurch gekennzeichnet, daß das Reaktionsgefäß eine Kolonne ist, die von Teilchen aus einem porösen Material vollständig angefüllt ist, die den genannten festen Träger darstellen.

3. Verfahren nach Anspruch 1 oder 2, dadurch gekennzeichnet, daß das Reaktionsgefäß die Form einer zylindrischen Kolonne hat.

4. Verfahren nach einem der Ansprüche 1 bis 3, dadurch gekennzeichnet, daß die feste Phase aus Teilchen besteht, die ihrerseits aus porösen Mikrokugeln bestehen.

5. Verfahren nach einem der Ansprüche 1 bis 4, dadurch gekennzeichnet, daß das Reaktionsgefäß bei einer Temperatur zwischen 30 und 60°C, vorzugsweise in der Größenordnung von 45°C, gehalten wird.

6. Verfahren nach einem der Ansprüche 1 bis 5, dadurch gekennzeichnet, daß man die Reagentien vor dem Einführen in das Reaktionsgefäß und gegebenenfalls vor dem Mischen derselben miteinander auf die Temperatur des Reaktionsgefäßes erwärmt.

7. Verfahren nach einem der Ansprüche 1 bis 6, dadurch gekennzeichnet, daß die Stufe der Schutzgruppenentfernung mit TFA in Dichlorethan durchgeführt wird.

8. Verfahren nach einem der Ansprüche 1 bis 7, dadurch gekennzeichnet, daß die Stufe der Oxidation mit Iod, gelöst in Essigsäure, und Pyridin durchgeführt wird.

9. Reaktionsgefäß zur Herstellung von Polynucleotiden nach dem Verfahren der Ansprüche 1 bis 8, das die Form einer Kolonne hat, die einen festen Träger enthält, durch den man die Lösungen der Reagentien und/oder Lösungsmittel zirkulieren läßt, dadurch gekennzeichnet, daß die feste Phase, die den festen Träger darstellt, in dem genannten Reaktionsgefäß in der Weise immobilisiert ist, daß die genannten Lösungen in der Kolonne und durch die genannte feste Phase hindurch in Form einer fortschreitenden Frontlinie so wandern, daß die aufeinanderfolgenden Lösungen jeder Stufe eines Synthesezyklus sich nicht oder nur wenig vermischen.

10. Reaktionsgefäß für die Verwendung in einem Verfahren zur Herstellung von Polynucleotiden auf einem festen Träger nach einem der Ansprüche 1 bis 8, dadurch gekennzeichnet, daß es aus einer zylindrischen Kolonne besteht, die mit Teilchen aus einem porösen Material vollständig angefüllt ist, die den festen Träger darstellen.

11. Reaktionsgefäß nach Anspruch 10, dadurch gekennzeichnet, daß die Teilchen poröse Mikrokugeln sind.

12. Reaktionsgefäß nach einem der Ansprüche 9 bis 11, dadurch gekennzeichnet, daß es thermostatisch bei einer Temperatur zwischen 30 und 60°C, vorzugsweise bei 45°C, gehalten werden kann.

13. Vorrichtung zur Synthese von Polynucleotiden auf einem festen Träger, die das thermostatisch geregelte Reaktionsgefäß nach einem der Ansprüche 9 bis 12 und einen thermostatisch geregelten Kollektor umfaßt, in dem die Reagentien vor ihrer Einführung in das Reaktionsgefäß gesammelt, auf die Temperatur des Reaktionsgefäßes erwärmt und dann miteinander gemischt werden.
